(19)

**Europäisches Patentamt**

**European Patent Office**

**Office européen des brevets**

(11) **EP 4 410 266 A1**

(12) **EUROPEAN PATENT APPLICATION**

(43) Date of publication:
**07.08.2024 Bulletin 2024/32**

(21) Application number: **23154154.1**

(22) Date of filing: **31.01.2023**

(51) International Patent Classification (IPC):
**A61K 8/19** *(2006.01)*　　　**A61K 8/43** *(2006.01)*
**A61K 8/41** *(2006.01)*　　　**A61Q 5/06** *(2006.01)*

(52) Cooperative Patent Classification (CPC):
**A61K 8/19; A61K 8/43; A61Q 5/065;**
A61K 2800/432; A61K 2800/88; A61K 2800/884

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Kao Corporation
Chuo-ku
Tokyo 103-8210 (JP)**

(72) Inventors:
• **NÖCKER, Bernd
64297 Darmstadt (DE)**

• **BREAKSPEAR, Steven
64297 Darmstadt (DE)**
• **LIPINSKI, Clarissa
64297 Darmstadt (DE)**
• **NEU, Anna
64297 Darmstadt (DE)**
• **GHIASI, Fariba
64297 Darmstadt (DE)**

(74) Representative: **Hoffmann Eitle
Patent- und Rechtsanwälte PartmbB
Arabellastraße 30
81925 München (DE)**

(54) **METHOD FOR DYEING OF KERATIN FIBERS**

(57) The present invention is directed to a method for dyeing keratin fibers, preferably human keratin fibers, more preferably human hair, comprising the steps of: i) applying to keratin fibers an aqueous composition A comprising: a) one or more earth alkaline metal salt(s), and/or b) one or more guanidine or guanidinium salt(s), ii) leaving composition A onto keratin fibers and rinsing-off composition A, iii) applying to keratin fibers a dyeing composition B having a pH in the range of 7 to 12 comprising: c) one or more non-ionic or anionic direct dye(s), and/or their salt(s), and/or their mixtures, d) one or more alkalizing agent(s), and/or their salt(s), and/or their mixtures, iv) leaving composition B onto keratin fibers and rinsing-off the keratin fibers, wherein the steps i) and ii) are carried out prior to step iii) and/or after step iv), wherein the total concentration of one or more compound(s) according to groups a) and/or b), and/or their mixtures, in composition A is 0.1% by weight or more, calculated to the total weight of composition A.

EP 4 410 266 A1

**Description**

**Field of the invention**

The present invention relates to a method and kit-of-parts for dyeing keratin fibers, preferably human keratin fibers, more preferably human hair. The dyeing method involves the application of a composition comprising earth alkaline metal salts and/or guanidine or guanidinium compounds as a pretreatment or posttreatment composition to the dyeing composition.

**Background of the invention**

Direct dyes are an attractive alternative to oxidative dyes, because the dyeing processes involving direct dyes do not necessarily require the mixing and application of oxidative compositions. Hence, the aforementioned dyeing processes are less damaging to keratin fibers.

A major drawback of with direct dyes is their unsatisfactory dyeing intensity on keratin fibers as well as their low wash fastness. The prior art has addressed these problems, but only partially delivered a satisfactory solution.

FR3060351, FR3060317, FR3060313, and JP2012126661 disclose oxidizing compositions for bleaching and dyeing of keratin fibers which comprise guanidine compounds. Guanidine compounds are disclosed to catalyze polymerization of oxidative dyes. However, the present invention is directed to direct dyes and has different technical effects as laid out below.

DE29722990, EP2883570, and EP2883571 disclose guanidine compounds in bleaching powder. However, the present invention is directed to an aqueous composition A.

WO2017/197099 discloses earth alkaline metal salts in pretreatment or posttreatment compositions to oxidative hair dyeing. However, the document is silent on the nature of direct dyes.

**Summary of the invention**

Therefore, the first object of the present invention is a method for dyeing keratin fibers, preferably human keratin fibers, more preferably human hair, comprising the steps of:

i) applying to keratin fibers an aqueous composition A comprising:

a) one or more earth alkaline metal salt(s), and/or

b) one or more guanidine or guanidinium salt(s),

ii) leaving composition A onto keratin fibers and rinsing-off composition A,

iii) applying to keratin fibers a dyeing composition B having a pH in the range of 7 to 12 comprising:

c) one or more non-ionic or anionic direct dye(s), and/or their salt(s), and/or their mixtures,

d) one or more alkalizing agent(s), and/or their salt(s), and/or their mixtures,

iv) leaving composition B onto keratin fibers and rinsing-off the keratin fibers,

wherein the steps i) and ii) are carried out prior to step iii) and/or after step iv),

wherein the total concentration of one or more compound(s) according to groups a) and/or b), and/or their mixtures, in composition A is 0.1 % by weight or more, calculated to the total weight of composition A.

The second object of the present invention is a kit-of-parts for dyeing of keratin fibers, preferably human keratin fibers, more preferably human hair comprising:

- an aqueous composition A as defined in any of the claims 1 to 13,

- a dyeing composition B as defined in any of the claims 1 to 13.

**Detailed description of the invention**

The present invention is directed to a method for dyeing keratin fibers, preferably human keratin fibers, more preferably human hair, comprising the steps of:

 i) applying to keratin fibers an aqueous composition A comprising:

   a) one or more earth alkaline metal salt(s), and/or

   b) one or more guanidine or guanidinium salt(s),

 ii) leaving composition A onto keratin fibers and rinsing-off composition A,

 iii) applying to keratin fibers a dyeing composition B having a pH in the range of 7 to 12 comprising:

   c) one or more non-ionic or anionic direct dye(s), and/or their salt(s), and/or their mixtures,

   d) one or more alkalizing agent(s), and/or their salt(s), and/or their mixtures,

 iv) leaving composition B onto keratin fibers and rinsing-off the keratin fibers,

   wherein the steps i) and ii) are carried out prior to step iii) and/or after step iv),

   wherein the total concentration of one or more compound(s) according to groups a) and/or b), and/or their mixtures, in composition A is 0.1 % by weight or more, calculated to the total weight of composition A.

This disclosed method also is a method for improving wash fastness of one or more non-ionic or anionic direct dye(s), and/or their salt(s), and/or their mixtures, on keratin fibers, preferably human keratin fibers, more preferably human hair.

**Pretreatment of composition B with composition A**

Hence, in one aspect of the present invention, composition A is a pretreatment composition to composition B. This aspect includes a method for dyeing keratin fibers, preferably human keratin fibers, more preferably human hair, comprising the steps of:

 i) applying to keratin fibers an aqueous composition A comprising:

   a) one or more earth alkaline metal salt(s), and/or

   b) one or more guanidine or guanidinium salt(s),

 ii) leaving composition A onto keratin fibers and rinsing-off composition A,

 iii) applying to keratin fibers a dyeing composition B having a pH in the range of 7 to 12 comprising:

   c) one or more non-ionic or anionic direct dye(s), and/or their salt(s), and/or their mixtures,

   d) one or more alkalizing agent(s), and/or their salt(s), and/or their mixtures,

 iv) leaving composition B onto keratin fibers and rinsing-off the keratin fibers,

   wherein the steps i) and ii) are carried out prior to step iii) and/or after step iv),
   wherein the total concentration of one or more compound(s) according to groups a) and/or b), and/or their mixtures, in composition A is 0.1% by weight or more, calculated to the total weight of composition A.

**Posttreatment of composition B with composition A**

In another aspect of the present invention, composition A is a posttreatment composition to composition B. This aspect includes a method for dyeing keratin fibers, preferably human keratin fibers, more preferably human hair, comprising the steps of:

iii) applying to keratin fibers a dyeing composition B having a pH in the range of 7 to 12 comprising:

e) one or more non-ionic or anionic direct dye(s), and/or their salt(s), and/or their mixtures,

f) one or more alkalizing agent(s), and/or their salt(s), and/or their mixtures,

iv) leaving the composition onto keratin fibers and rinsing-off the keratin fibers,

i) applying to keratin fibers an aqueous composition A comprising:

a) one or more earth alkaline metal salt(s), and/or

b) one or more guanidine or guanidinium salt(s),

ii) leaving composition A onto keratin fibers and rinsing-off composition A,
wherein the total concentration of one or more compounds according to groups a) and/or b), and/or their mixtures, in composition A, is 0.1% by weight or more, calculated to the total weight of composition A.

**Composition A**

Irrespective of the fact that composition A may be a pretreatment or posttreatment composition to composition B, the following disclosure relates to both aspects of executing the inventive method.
Composition A is an aqueous composition. 'Aqueous' within the meaning of the present invention denotes a composition that comprises water at 50% by weight or more, preferably 60% by weight or more, more preferably 75% by weight or more, calculated to the total weight of composition A.
It is preferred from the viewpoint of dyeing intensity and cosmetic safety that the pH of composition A is 2 or more, preferably 3 or more, more preferably 4 or more.
It is further preferred from the viewpoint of cosmetic safety the pH of composition A is 12 or less, preferably 11 or less, more preferably 10 or less.
For attaining the above-mentioned effects, it is preferred that the pH of composition A is in the range of 2 to 12, preferably in the range of 3 to 11, more preferably in the range of 4 to 10.
It is preferred from the viewpoint of dyeing intensity and wash fastness that one or more compound(s) according to group a) is/are magnesium sulfate, magnesium chloride, magnesium nitrate, magnesium carbonate, magnesium bicarbonate, magnesium phosphate, calcium chloride, calcium nitrate, calcium bicarbonate, and/or their mixtures.
It is preferred from the viewpoint of dyeing intensity and wash fastness that one or more compound(s) according to group b) is/are selected from guanidine carbonate, guanidine hydrochloride, guanidine sulfate, guanidine phosphate, and/or their mixtures, preferably it is guanidine sulfate.
It is further preferred from the viewpoint of dyeing intensity and wash fastness that the total concentration of compound(s) according to groups a) and/or b), and/or their mixtures, is 0.1% by weight or more, preferably 0.5% by weight or more, still more preferably 1% by weight or more, calculated to the total weight of composition A.
It is preferred from the viewpoint of cosmetic safety that the total concentration of compound(s) according to groups a) and/or b), and/or their mixtures, is 30% by weight or less, preferably 20% by weight or less, more preferably 15% by weight or less, still more preferably 10% by weight or less, calculated to the total weight of composition A.
For attaining the above-mentioned effects, it is preferred that the total concentration of compound(s) according to groups a) and/or b), and/or their mixtures, is in the range of 0.1% to 30% by weight, preferably in the range of 0.1% to 20% by weight, more preferably in the range of 0.5% to 15% by weight, still more preferably in the range of 1% to 10% by weight, calculated to the total weight of composition A.
It is further preferred from the viewpoint of cosmetic acceptance and dyeing intensity that composition A comprises less than 1% by weight, preferably less than 0.5% by weight, more preferably less than 0.1% by weight of oxidative hair dyes and direct hair dyes, calculated to the total weight of composition A, more preferably composition A is free of oxidative hair dyes and direct hair dyes.
It is further preferred from the viewpoint of cosmetic safety and dyeing intensity that composition A is comprises less than 1% by weight, preferably less than 0.5% by weight, more preferably less than 0.1% by weight of oxidizing agents and reducing agents, calculated to the total weight of composition A, more preferably composition A is free of oxidizing agents and reducing agents.

Compositions A and/or B may comprise one or more surfactant(s), preferably selected from anionic, non-ionic, cationic and/or amphoteric/zwitterionic surfactants, and/or their mixtures, from the viewpoint of customer convenience.

Preferably, the anionic surfactants are selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, from the viewpoint of their emulsion stabilization properties.

Suitable alkyl sulfate or preferably ethoxylated alkyl ether sulfate surfactant or mixtures thereof have an alkyl chain length of $C_{10}$ to $C_{22}$.

Suitable example alkyl sulfate surfactants are lauryl sulfate, myristyl sulfate, olel sulfate, and behenyl sulfate.

Suitable example alkyl ether sulfate surfactants are laureth sulfates, coceth sulfate, pareth sulfate, capryleth sulphate, myreth sulfate, oleth sulfate, deceth sulfate, trideceth sulfate, coco sulphate, $C_{10}$-$C_{16}$ alkyl sulphate, $C_{11}$-$C_{15}$ alkyl sulphate, $C_{12}$-$C_{18}$ alkyl sulphate, $C_{12}$-$C_{15}$ alkyl sulphate, $C_{12}$-$C_{16}$ alkyl sulphate, $C_{12}$-$C_{13}$ alkyl sulfate, lauryl sulphate, myrystyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Cations for all of the above-mentioned surfactants may be selected from sodium, potassium, magnesium and/or ammonium.

Suitable non-ionic surfactants are selected from alkyl polyglycosides, ethoxylated triglycerides, ethoxylated fatty alcohols, and/or their mixtures.

Suitable examples of alkyl polyglycosides are decyl glucoside, lauryl glucoside, and coco glucoside.

Further suitable nonionic surfactants are ethoxylated triglycerides. Well-known and commonly used examples are ethoxylated castor oil such as PEG-40 hydrogenated castor oil or and PEG-60 hydrogenated castor oil.

Suitable examples of ethoxylated fatty alcohols are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine, and/or their salt(s).

Further suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable examples of cationic surfactants are cetyl trimethyl ammonium chloride, stearyl trimonium chloride, dipalmitoyl dimonium chloride, distearyl dimethyl ammonium chloride, stearamidopropyl trimethyl ammonium chloride, dioleoylethyl dimethyl ammonium methosulfate, dioleoylethyl hydroxyethylmonium methosulfate, behenyl trimethyl ammonium chloride.

Preferably, the total concentration of surfactants in compositions A and or B is 0.05% by weight or more, more preferably 0.1% by weight or more, further more preferably 0.2% by weight or more, calculated to the total weight of each of the compositions A and/or B, from the viewpoint of dyeing intensity.

Preferably, the total concentration of surfactants in composition A and/or B is 15% by weight or less, more preferably 10% by weight or less, still more preferably 5% by weight or less, calculated to the total weight of each of the compositions A and/or B, from the viewpoint of stability.

For attaining the above-mentioned effect, preferably the total concentration of surfactants in compositions A and/or B is in the range of 0.05% to 15% by weight, preferably 0.1% to 10% by weight, more preferably 0.2% to 5% by weight, calculated to the total weight of each of the compositions A and/or B.

The preferred surfactants are anionic surfactants and/or non-ionic surfactants from the viewpoint of customer convenience. For example, composition A may have the form of a hair mask or shampoo comprising these surfactants.

Suitably, composition A comprises one or more lipophilic compound(s), preferably selected from branched or linear, saturated or unsaturated $C_{12}$-$C_{22}$ fatty alcohols, esters of branched or linear $C_3$-$C_{12}$ alcohols with linear or branched, saturated or unsaturated $C_8$-$C_{22}$ fatty acids, and petrolatum-based products, and/or their mixtures, from the viewpoint of emulsion preparation.

Preferably, the petrolatum-based products are selected from mineral oil, paraffinum perliquidum, paraffinum subliquidum, and/or paraffinum solidum, and/or their mixtures, from the viewpoint of their conditioning properties.

Suitably, branched or linear, saturated or unsaturated $C_{12}$-$C_{22}$ fatty alcohols are lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures. Such a mixture is cetearyl alcohol comprising stearyl alcohol and cetyl alcohol.

The most preferred lipophilic compounds are cetearyl alcohol and/or mineral oil, from the viewpoint of cosmetic compatibility.

It is preferred that the total concentration of lipophilic compounds in compositions A and/or B is 0.5% by weight or more, preferably 1% by weight or more, more preferably 2% by weight or more, calculated to the total weight of each of the compositions A and/or B, from the viewpoint of enhancing color intensity on keratin fibers.

It is preferred that the total concentration of lipophilic compounds in compositions A and/or B is 20% by weight or less, preferably 15% by weight or less, more preferably 12% by weight or less, calculated to the total weight of each of the compositions A and/or B, from the viewpoint of enhancing color intensity on keratin fibers and formulating a stable cosmetic composition.

For attaining the above-mentioned effects, it is preferred that the total concentration of lipophilic compounds in compositions A and/or B is in the range of 0.5% to 20% by weight, preferably in the range of 1% to 15% by weight, more preferably in the range of 2% to 12% by weight, calculated to the total weight of each of the compositions A and/or B.

Suitably, compositions A and/or B may comprise one or more thickening polymers, preferably a thickening polymer which is able to produce a viscosity of 500 mPas in a 1% by weight aqueous solution within the desired pH range of 2 to less than 12.

Suitable polymers are, for example, xanthan gum, dehydroxanthan gum, methyl cellulose, ethyl cellulose, hydroxymethyl cellulose, and hydroxyethylcellulose, and/or their mixtures.

It is preferred from the viewpoint of viscosity and convenience that the total concentration of thickening polymers of compositions A and/or B is 0.1 % by weight or more, more preferably 0.2% by weight or more, further more preferably 0.5% by weight or more, calculated to the total weight of each of the compositions A and/or B.

It is preferred from the viewpoint of viscosity and convenience that the total concentration of thickening polymers of compositions A and/or B is 10% by weight or less, more preferably 8% by weight or less, further more preferably 5% by weight or less, calculated to the total weight of each of the compositions A and/or B.

For attaining the above-mentioned effects, it is preferred that the total concentration of thickening polymers of compositions A and/or B is in the range of 0.1% to 10% by weight, preferably in the range of 0.2% to 8% by weight, more preferably in the range of 0.5% to 5% by weight, calculated to the total weight of each of the compositions A and/or B.

It is to be noted that the skilled person may combine lipophilic compounds and thickening polymers. With both measures, it is possible to thicken the compositions A and/or B to achieve the desired technical effect. In this case, the skilled person will select appropriate ratios of lipophilic compounds and thickening polymers.

**Composition B**

It is preferred from the viewpoint of wash fastness and dyeing intensity that one or more compound(s) according to group c) of composition B is/are selected from Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Acid Red 52, DC Violet 2, DC Red 33, DC Orange 4, DC Red 27, DC Yellow 10, 2-Amino-6-chloro-4-nitrophenol, HC Blue 18, HC Red 18, HC Yellow 16, and/or their salt(s), and/or their mixtures, preferably it/they is/are selected from HC Blue 18, HC Red 18, HC Yellow 16, 2-Amino-6-chloro-4-nitrophenol, Acid Yellow 1, and/or Disperse Black 9, an/or their salt(s), and/or their mixtures, more preferably it/they is/are selected from HC Blue 18, HC Red 18, HC Yellow 16, and/or their salt(s), and/or their mixtures.

It is preferred from the viewpoint of dyeing intensity that the total concentration of one or more compound(s) according to group c) in composition B is 0.01% by weight or more, preferably 0.05% by weight or more, still more preferably 0.1% by weight or more, calculated to the total weight of composition B.

It is preferred from the viewpoint of cosmetic acceptance that the total concentration of one or more compound(s) according to group c) in composition B is 10% by weight or less, preferably 8% by weight or less, still more preferably 5% by weight or less, calculated to the total weight of composition B.

For attaining the above-mentioned effects, it is preferred that the total concentration of one or more compound(s) according to group c) in composition B is in the range of 0.01% to 10% by weight, preferably in the range of 0.05% to 8% by weight, more preferably in the range of 0.1% to 5% by weight, calculated to the total weight of composition B.

It is further preferred from the viewpoint of dyeing intensity that one or more compound(s) according to group d) in composition B is selected from ammonia and/or its salt(s), alkyl and/or alkanolamine(s) and/or its/their salt(s), preferably it/they is/are selected from monoethanolamine, diethanolamine, triethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethan and/or its/their salt(s), and/or their mixtures, more preferably it/they is/are selected from monoethanolamine, 2-amino-2-methylpropanol, tris-(hydroxymethyl)-aminomethane and/or its/their salt(s), and/or their mixtures.

It is preferred from the viewpoint of dyeing intensity that the total concentration of compound(s) according to group d) in composition B, preferably the total concentration of one or more alkyl amine or alkanolamine(s) and/or its/their salt(s) and/or ammonia and/or its salt(s) in composition B, is 0.1% by weight or more, preferably 0.25% by weight or more, more preferably 0.5% by weight or more, calculated to the total weight of composition B.

It is preferred from the viewpoint of cosmetic safety that the total concentration of compound(s) according to group d) in composition B, preferably the total concentration of one or more alkyl amine or alkanolamine(s) and/or its/their salt(s) and/or ammonia and/or its salt(s) in composition B, is 40% by weight or less, preferably 30% by weight or less, more preferably 25% by weight or less, calculated to the total weight of composition B.

For attaining the above-mentioned effects, it is preferred that the total concentration of compound(s) according to group d) in composition B, preferably the total concentration of one or more alkyl amine or alkanolamine(s) and/or its/their salt(s) and/or ammonia and/or its salt(s) in composition B, is in the range of 0.1% to 40% by weight, preferably in the range of 0.25% to 30% by weight, more preferably in the range of 0.5% to 25% by weight, calculated to the total weight of composition B.

**Further definition of method steps**

It is further preferred from the viewpoint of dyeing intensity that the leave-on time for composition A in step ii) is 1 min or more, preferably 2 min or more, more preferably 5 min or more, further more preferably 10 min or more.

It is further preferred from the viewpoint of customer convenience that the leave-on time of composition A in step ii) is 72 h or less, preferably 24 h or less, more preferably 12 h or less, further more preferably 60 min or less.

For attaining the above-mentioned effects, it is preferred that the leave-on time of composition A in step ii) is in the range of 1 min to 72 h, preferably in the range of 2 min to 24 h, more preferably in the range of 5 min to 12 h, further more preferably in the range of 10 min to 60 min.

It is further more preferred from the viewpoint of dyeing intensity that composition B comprises less than 1% by weight, preferably less than 0.5% by weight, more preferably less than 0.1% by weight of compound(s) a) and b), calculated to the total weight of composition B, more preferably composition B is free of compound(s) a) and b).

It is further preferred from the viewpoint of dyeing intensity that the leave-on time for composition B in step iv) is 1 min or more, preferably 2 min or more, more preferably 5 min or more, further more preferably 10 min or more.

It is further preferred from the viewpoint of customer convenience that the leave-on time of composition B in step iv) is 72 h or less, preferably 24 h or less, more preferably 12 h or less, further more preferably 60 min or less.

For attaining the above-mentioned effects, it is preferred that the leave-on time of composition B in step iv) is in the range of 1 min to 72 h, preferably in the range of 2 min to 24 h, more preferably in the range of 5 min to 12 h, further more preferably in the range of 10 min to 60 min.

**Kit-of-parts**

The present invention is also directed to a kit-of-parts for dyeing of keratin fibers, preferably human keratin fibers, more preferably human hair comprising:

- an aqueous composition A as defined above,

- a dyeing composition B as defined above.

Preferably, the kit-of-parts comprises

- an aqueous composition A comprising one or more earth alkaline metal(s) as compound(s) according to group b) at a total concentration of 0.1% by weight or more, calculated to the total weight of composition A,

- a composition B comprising:

c) one or more non-ionic or anionic direct dye(s), and/or their salt(s), and/or their mixtures,

d) one or more alkalizing agent(s), and/or their salt(s), and/or their mixtures.

The following examples are to illustrate the present invention and not to limit it.

## EXAMPLES

Table 1 – Pretreatment with composition B

| Ingredients | | Comp. 1 | Comp. 2 | Comp. 3 | Comp. 4 | Comp. 5 | Comp. 6 | Comp. 7 | Comp. 8 | Inv. 1 | Inv. 2 | Inv. 3 | Inv. 4 |
|---|---|---|---|---|---|---|---|---|---|---|---|---|---|
| Composition A | GnHCl | - | - | - | - | - | - | 1.0 | 1.0 | - | - | 1.0 | 1.0 |
| | GnSO4 | - | - | - | - | 1.0 | 1.0 | - | - | 1.0 | 1.0 | - | - |
| | NaOH/HCl ad pH | 4.0 | 9.0 | 4.0 | 9.0 | 4.0 | 9.0 | 4.0 | 9.0 | 4.0 | 9.0 | 4.0 | 9.0 |
| | Water | Ad 100.0 | | | | | | | | | | | |
| Composition B | HC Yellow 16 | 0.25 | 0.25 | - | - | - | - | | | 0.25 | 0.25 | 0.25 | 0.25 |
| | Basic Yellow 87 | - | - | 0.25 | 0.25 | 0.25 | 0.25 | 0.25 | 0.25 | - | - | - | - |
| | Monoethanolamine | 2.5 | | | | | | | | | | | |
| | Xanthan gum | 2.0 | | | | | | | | | | | |
| | NaOH/HCl ad pH | 9.5 | | | | | | | | | | | |
| | Water | Ad 100.0 | | | | | | | | | | | |
| Evaluation | Color upon dyeing (ΔE) | 73.9 | 75.8 | 62.1 | 64.7 | 64.5 | 62.2 | 60.7 | 60.1 | 77.4 | 77.4 | 76.4 | 76.8 |
| | Color upon ultrasonic treatment (ΔE) | 57.2 | 57.9 | 39.5 | 41.0 | 37.7 | 39.7 | 36.7 | 33.2 | 65.5 | 65.7 | 64.2 | 63.3 |
| | Color loss upon ultrasonic treatment (ΔΔE) | 16.7 | 17.9 | 22.6 | 23.7 | 26.8 | 22.5 | 24.0 | 26.9 | 11.9 | 11.7 | 12.2 | 13.5 |

HC Yellow 16 is a representative compound for anionic direct dyes at pH 9 and non-ionic direct dyes at pH 4. Inventive examples 1-4 compare directly to comparative examples 1 and 2, as the comparative examples lack the guanidine compounds. The loss on color of the inventive examples was decreased by at least 3 ΔE units in presence of guanidine compounds. Basic Yellow 87 as a representative of cationic direct dyes showed a higher color loss in comparison to HC Yellow 16 (comparative examples 3-4). Moreover, the presence of guanidine compounds increased color loss in some cases for Basic Yellow 87 (comparative examples 5-8), thereby indicating no or a detrimental effect of the presence of guanidine compounds to cationic direct dyes with respect to wash fastness.

## Table 2 – Pretreatment with composition B

| Ingredients | | Comp. 17 | Inv. 9 | Inv. 10 | Comp. 18 | Inv. 11 | Inv. 12 | Comp. 19 | Inv. 13 | Comp. 20 | Inv. 14 |
|---|---|---|---|---|---|---|---|---|---|---|---|
| Composition A | GnSO4 | - | - | - | - | - | - | - | 20.0 | - | 5.0 |
| | CaCl2 | - | 1.0 | 20.0 | - | 1.0 | 20.0 | - | - | - | - |
| | NaOH/HCl ad pH | 7.0 | 7.0 | 7.0 | 7.0 | 7.0 | 7.0 | 4.0 | 4.0 | 10.0 | 10.0 |
| | Water | Ad 100.0 | | | | | | | | | |
| Composition B | HC Yellow 16 | 0.25 | 0.25 | 0.25 | - | - | - | - | | - | - |
| | HC Blue 18 | - | - | - | 0.25 | 0.25 | 0.25 | 0.25 | 0.25 | - | - |
| | HC Red 18 | - | - | - | - | - | - | - | - | 0.25 | 0.25 |
| | Monoethanolamine | 2.5 | | | | | | | | | |
| | Xanthan gum | 2.0 | | | | | | | | | |
| | NaOH/HCl ad pH | 9.5 | | | | | | | | | |
| | Water | Ad 100.0 | | | | | | | | | |
| Evaluation | Color upon dyeing ($\Delta E$) | 68.3 | 76.0 | 75.2 | 71.9 | 73.1 | 73.6 | 73.3 | 73.6 | 72.5 | 73.4 |
| | Color upon ultrasonic treatment ($\Delta E$) | 46.1 | 59.8 | 59.4 | 59.3 | 64.8 | 68.7 | 59.1 | 63.0 | 71.2 | 73.4 |
| | Color loss upon ultrasonic treatment ($\Delta\Delta E$) | 22.2 | 16.2 | 15.8 | 12.6 | 5.4 | 4.9 | 14.2 | 10.6 | 1.2 | 0.0 |

## Table 3 – Posttreatment with composition B

| Ingredients | | Comp. 9 | Comp. 10 | Comp. 11 | Comp. 12 | Comp. 13 | Comp. 14 | Comp. 15 | Comp. 16 | Inv. 5 | Inv. 6 | Inv. 7 | Inv. 8 |
|---|---|---|---|---|---|---|---|---|---|---|---|---|---|
| Composition A | GnHCl | - | - | - | - | - | - | 1.0 | 1.0 | - | - | 1.0 | 1.0 |
| | GnSO4 | - | - | - | - | 1.0 | 1.0 | - | - | 1.0 | 1.0 | - | - |
| | NaOH/HCl ad pH | 4.0 | 9.0 | 4.0 | 9.0 | 4.0 | 9.0 | 4.0 | 9.0 | 4.0 | 9.0 | 4.0 | 9.0 |
| | Water | Ad 100.0 | | | | | | | | | | | |
| Composition B | HC Yellow 16 | 0.25 | 0.25 | - | - | - | - | | | 0.25 | 0.25 | 0.25 | 0.25 |
| | Basic Yellow 87 | - | - | 0.25 | 0.25 | 0.25 | 0.25 | 0.25 | 0.25 | - | - | - | - |
| | Monoethanolamine | 2.5 | | | | | | | | | | | |
| | Xanthan gum | 2.0 | | | | | | | | | | | |
| | NaOH/HCl ad pH | 9.5 | | | | | | | | | | | |
| | Water | Ad 100.0 | | | | | | | | | | | |
| Evaluation | Color upon dyeing (ΔE) | 75.3 | 74.8 | 66.1 | 61.7 | 60.6 | 57.3 | 57.3 | 58.2 | 74.8 | 74.6 | 75.6 | 74.7 |
| | Color upon ultrasonic treatment (ΔE) | 57.7 | 58.3 | 36.5 | 40.8 | 38.3 | 37.0 | 34.2 | 34.6 | 60.5 | 61.4 | 61.2 | 61.0 |
| | Color loss upon ultrasonic treatment (ΔΔE) | 17.6 | 16.5 | 29.6 | 20.9 | 22.3 | 20.3 | 23.1 | 23.6 | 14.3 | 13.2 | 14.4 | 13.7 |

Applying composition A as posttreatment composition revealed a similar trend as for pretreatment. Wash fastness of the inventive compositions 5-8 was in all cases improved with respect to comparative compositions 9 and 10 not comprising guanidine compounds. The effect was not present for Basic Yellow 87 as a representative cationic direct dye.

**Methods**

**Tables 1 and 2 - Pretreatment with composition B**

Goat hair streaks (11 cm long, 1 g per streak) were immersed in 100 g of the compositions B as described in table 1 for 30 min at 40°C. Then the hair streaks were rinsed-off with tap water for 10 s and blow-dried. For coloring the hair streaks, compositions A as described in table 1 were mixed with a composition comprising 6% hydrogen peroxide in a weight ratio of 1:1 to prepare ready-to-use mixtures. The ready-to-use mixtures had a pH of 9.7. 4 g of the ready-to-use mixtures were applied to the hair streaks and left for 30 min at 40°C. Then the hair streaks were rinsed off with tap water and blow-dried. Colorimetric data were obtained with a color-difference meter (Datacolor Mercury Check II Plus) in the CIE colorimetric system (L*,a*,b*). Color differences (ΔE) were calculated by the following formula.

$$\Delta E = \sqrt{(L_1^* - L_0^*)^2 + (a_1^* - a_0^*)^2 + (b_1^* - b_0^*)^2}$$

**Table 3 - Posttreatment with composition B**

Goat hair streaks (11 cm long, 1g per bundle) were applied 4 g of ready-to-use mixtures prepared by mixing compositions A as described in table 2 with a composition comprising 6% hydrogen peroxide in a weight ratio of 1:1. The ready-to-use mixtures had a pH of 9.7. The ready-to-use mixtures were left for 30 min at 40°C. The hair streaks were rinsed off with tap water and blow-dried. Then the streaks were immersed in 100 g of the compositions B as described in table 2 for 30 min at 40°C. The hair streaks were rinsed-off with tap water for 10 s and blow-dried. Colorimetric data were recorded as explained for the pretreatment.

**Wash fastness**

The dyed and treated hair streaks were placed in an ultrasonic bath comprising an aqueous solution of 3.5% by weight of sodium laureth sulfate at pH 5.6. The hair streaks were treated with ultrasound for 30 min at 40°C, then they were removed from the bath and washed with lukewarm tap water. After treatment, the hairstreaks were blow-dried and the remaining color was measured (L*,a*,b*). ΔΔE was calculated as the color difference between ultrasound-treated hair and freshly colored hair.

**Claims**

1.  A method for dyeing keratin fibers, preferably human keratin fibers, more preferably human hair, comprising the steps of:

    i) applying to keratin fibers an aqueous composition A comprising:

    a) one or more earth alkaline metal salt(s), and/or
    b) one or more guanidine or guanidinium salt(s),

    ii) leaving composition A onto keratin fibers and rinsing-off composition A,
    iii) applying to keratin fibers a dyeing composition B having a pH in the range of 7 to 12 comprising:

    c) one or more non-ionic or anionic direct dye(s), and/or their salt(s), and/or their mixtures,
    d) one or more alkalizing agent(s), and/or their salt(s), and/or their mixtures,

    iv) leaving composition B onto keratin fibers and rinsing-off the keratin fibers,

    wherein the steps i) and ii) are carried out prior to step iii) and/or after step iv),
    wherein the total concentration of one or more compound(s) according to groups a) and/or b), and/or their mixtures, in composition A is 0.1% by weight or more, calculated to the total weight of composition A.

2.  The method according to claim 1 **characterized in that** the pH of composition A is in the range of 2 to 12, preferably in the range of 3 to 11, more preferably in the range of 4 to 10.

3. The method according to any of the claims 1 and/or 2 **characterized in that** one or more compound(s) according to group a) is/are magnesium sulfate, magnesium chloride, magnesium nitrate, magnesium carbonate, magnesium bicarbonate, magnesium phosphate, calcium chloride, calcium nitrate, calcium bicarbonate, and/or their mixtures.

4. The method according to any of the preceding claims **characterized in that** one or more compound(s) according to group b) is/are selected from guanidine carbonate, guanidine hydrochloride, guanidine sulfate, guanidine phosphate, and/or their mixtures, preferably it is guanidine sulfate.

5. The method according to any of the preceding claims **characterized in that** the total concentration of compound(s) according to groups a) and/or b), and/or their mixtures, is in the range of 0.1% to 30% by weight, preferably in the range of 0.1% to 20% by weight, more preferably in the range of 0.5% to 15% by weight, still more preferably in the range of 1% to 10% by weight, calculated to the total weight of composition A.

6. The method according to any of the preceding claims **characterized in that** composition A comprises less than 1% by weight, preferably less than 0.5% by weight, more preferably less than 0.1% by weight of oxidative hair dyes and direct hair dyes, calculated to the total weight of composition A, more preferably composition A is free of oxidative hair dyes and direct hair dyes.

7. The method according to any of the preceding claims **characterized in that** composition A is comprises less than 1% by weight, preferably less than 0.5% by weight, more preferably less than 0.1% by weight of oxidizing agents and reducing agents, calculated to the total weight of composition A, more preferably composition A is free of oxidizing agents and reducing agents.

8. The method according to any of the preceding claims **characterized in that** one or more compound(s) according to group c) is/are selected from Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Acid Red 52, DC Violet 2, DC Red 33, DC Orange 4, DC Red 27, DC Yellow 10, 2-Amino-6-chloro-4-nitrophenol, HC Blue 18, HC Red 18, HC Yellow 16, and/or their salt(s), and/or their mixtures, preferably it/they is/are selected from HC Blue 18, HC Red 18, HC Yellow 16, 2-Amino-6-chloro-4-nitrophenol, Acid Yellow 1, and/or Disperse Black 9, an/or their salt(s), and/or their mixtures, more preferably it/they is/are selected from HC Blue 18, HC Red 18, HC Yellow 16, and/or their salt(s), and/or their mixtures.

9. The method according to any of the preceding claims **characterized in that** the total concentration of one or more compound(s) according to group c) is in the range of 0.01% to 10% by weight, preferably in the range of 0.05% to 8% by weight, more preferably in the range of 0.1% to 5% by weight, calculated to the total weight of composition B.

10. The method according to any of the preceding claims **characterized in that** one or more compound(s) according to group d) is selected from ammonia and/or its salt(s), alkyl and/or alkanolamine(s) and/or its/their salt(s), preferably it/they is/are selected from monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethan and/or its/their salt(s), and/or their mixtures, more preferably it/they is/are selected from monoethanolamine, 2-amino-2-methylpropanol, tris-(hydroxymethyl)-aminomethane and/or its/their salt(s), and/or their mixtures.

11. The method according to any of the preceding claims **characterized in that** the total concentration of compound(s) according to group d), preferably the total concentration of one or more alkyl amine or alkanolamine(s) and/or its/their salt(s) and/or ammonia and/or its salt(s), is in the range of 0.1% to 40% by weight, preferably in the range of 0.25% to 30% by weight, more preferably in the range of 0.5% to 25% by weight, calculated to the total weight of the composition B.

12. The method according to any of the preceding claims **characterized in that** the leave-on time in step ii) is in the range of 1 min to 72 h, preferably in the range of 2 min to 24 h, more preferably in the range of 5 min to 12 h, further more preferably in the range of 10 min to 60 min.

13. The method according to any of the preceding claims **characterized in that** composition B comprises less than 1% by weight, preferably less than 0.5% by weight, more preferably less than 0.1% by weight of compound(s) a) and b), calculated to the total weight of composition B, more preferably composition B is free of compound(s) a) and b).

14. A kit-of-parts for dyeing of keratin fibers, preferably human keratin fibers, more preferably human hair, comprising:

    - an aqueous composition A as defined in any of the claims 1 to 13,
    - a dyeing composition B as defined in any of the claims 1 to 13.

15. The kit-of-parts according to claim 14 **characterized in that** the kit comprises

    - a composition A comprising one or more earth alkaline metal(s) as compound(s) according to group a) at a total concentration of 0.1% by weight or more, calculated to the total weight of composition A,
    - a composition B comprising:

        c) one or more non-ionic or anionic direct dye(s), and/or their salt(s), and/or their mixtures,
        d) one or more alkalizing agent(s), and/or their salt(s), and/or their mixtures.

Europäisches
Patentamt

European
Patent Office

Office européen
des brevets

# EUROPEAN SEARCH REPORT

Application Number

EP 23 15 4154

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/323326 A1 (NÖCKER BERND [DE] ET AL) 13 October 2022 (2022-10-13) | 14 | INV.<br>A61K8/19 |
| Y | * paragraphs [0001], [0003], [0145], [0153]; examples 4-7, 12, 13, 15-25 * | 1-15 | A61K8/43<br>A61K8/41<br>A61Q5/06 |
| Y | US 2020/188262 A1 (NÖCKER BERND [DE] ET AL) 18 June 2020 (2020-06-18)<br>* paragraph [0001]; claim 1 * | 1-15 | |
| Y | US 2002/189031 A1 (JAVET MANUELA [CH] ET AL) 19 December 2002 (2002-12-19)<br>* paragraphs [0001], [0004], [0007], [0037]; examples 1.1-1.5; table 2 * | 1-15 | |

TECHNICAL FIELDS
SEARCHED      (IPC)

A61K
A61Q

The present search report has been drawn up for all claims

| Place of search | Date of completion of the search | Examiner |
|---|---|---|
| Munich | 13 July 2023 | Bader, Karl Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 15 4154**

This annex lists the patent family members relating to the patent documents cited in the above-mentioned European search report.
The members are as contained in the European Patent Office EDP file on
The European Patent Office is in no way liable for these particulars which are merely given for the purpose of information.

**13-07-2023**

| Patent document cited in search report | | Publication date | Patent family member(s) | | Publication date |
|---|---|---|---|---|---|
| US 2022323326 | A1 | 13-10-2022 | BR 112022002667 | A2 | 10-05-2022 |
| | | | CN 114222557 | A | 22-03-2022 |
| | | | EP 4013375 | A1 | 22-06-2022 |
| | | | JP 2022544960 | A | 24-10-2022 |
| | | | TW 202114622 | A | 16-04-2021 |
| | | | US 2022323326 | A1 | 13-10-2022 |
| | | | WO 2021028532 | A1 | 18-02-2021 |
| US 2020188262 | A1 | 18-06-2020 | EP 3342464 | A1 | 04-07-2018 |
| | | | EP 3562557 | A1 | 06-11-2019 |
| | | | US 2020188262 | A1 | 18-06-2020 |
| | | | WO 2018122090 | A1 | 05-07-2018 |
| US 2002189031 | A1 | 19-12-2002 | AU 4836101 | A | 14-01-2002 |
| | | | DE 10032752 | A1 | 31-01-2002 |
| | | | EP 1210061 | A1 | 05-06-2002 |
| | | | JP 2004501943 | A | 22-01-2004 |
| | | | US 2002189031 | A1 | 19-12-2002 |
| | | | WO 0202062 | A1 | 10-01-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 3060351
- FR 3060317
- FR 3060313
- JP 2012126661 B

- DE 29722990
- EP 2883570 A
- EP 2883571 A
- WO 2017197099 A